# EUROPEAN PATENT APPLICATION

(11) **EP 3 488 787 A1**
(43) Date of publication of application: **29.05.2019**
(21) Application number: 17203727.7
(22) Date of filing: 27.11.2017
(51) Int. Cl.: A61B 8/08, A61B 8/12, A61B 8/00, A61B 5/145, A61B 5/021

(54) **ULTRASOUND IMAGE GENERATION SYSTEM FOR GENERATING AN INTRAVASCULAR ULTRASOUND IMAGE**

(71) Applicant: Koninklijke Philips N.V., 5656 AE Eindhoven (NL)
(72) Inventor: WISSEL, Tobias, 5656 AE Eindhoven (NL); WEBER, Frank Michael, 5656 AE Eindhoven (NL)
(74) Representative: de Haan, Poul Erik

(57) **Abstract**

The invention relates to the field of intravascular ultrasound (IVUS) imaging. IVUS images of a vessel 6 are provided, which have been determined based on IVUS signals acquired at different acquisition locations 9, 10 along the length of the vessel and at different acquisition times. Moreover, pressure values are provided, which are indicative of the pressure within the vessel at the acquisition times, wherein a combined IVUS image is generated by combining provided IVUS images that have been determined based on IVUS signals acquired at acquisition times at which the pressure values differ by less than a predefined deviation threshold from a reference pressure value. This use of the pressure within the vessel as a selection criterion for combining IVUS images can ensure that only IVUS images are combined, which correspond to a same vessel wall motion state, thereby reducing artifacts in the combined IVUS image.

## Description

### FIELD OF THE INVENTION

The invention relates to an ultrasound image generation system, method and computer program for generating an intravascular ultrasound image.

### BACKGROUND OF THE INVENTION

Ultrasound image generation systems are known that acquire several cross-sectional intravascular ultrasound (IVUS) images of a vessel during a pullback of an ultrasound signals acquisition element, that use an electrocardiogram (ECG) for selecting the cross-sectional IVUS images which have been acquired at a same cardiac phase and that combine adjacent selected cross-sectional IVUS images for generating a longitudinal IVUS view of the vessel. Since the ECG is not a good measure for movements of the vessel walls, the combined IVUS images likely correspond to different vessel wall motion states, which can cause image artifacts in the longitudinal IVUS view of the vessel.

### SUMMARY OF THE INVENTION

It is an object of the present invention to provide an ultrasound image generation system, method and computer program that allow for a generation of a longitudinal IVUS view of a vessel having an improved image quality.

In a first aspect of the present invention, an ultrasound image generation system for generating an intravascular ultrasound image is presented, wherein the ultrasound image generation system comprises:
- an ultrasound images providing unit for providing IVUS images of a vessel, which have been determined based on IVUS signals acquired at different acquisition locations along the length of the vessel and at different acquisition times,
- an acquisition locations providing unit for providing the different acquisition locations at which the IVUS signals have been acquired,
- a pressure values providing unit for providing intravascular pressure values being indicative of the pressure within the vessel at the acquisition times,
- an ultrasound images combining unit for generating a combined ultrasound image by combining provided IVUS images, which have been determined based on IVUS signals acquired at acquisition times at which the pressure values differ by less than a predefined deviation threshold from a reference pressure value, depending on the acquisition locations.

Since the ultrasound images combining unit is adapted to generate the combined ultrasound image by combining provided IVUS images which have been acquired at acquisition times at which the pressure values differ by less than a predefined deviation threshold from a reference pressure value, i.e. since IVUS images corresponding to similar pressure values are combined, and since the intravascular pressure is a good measure for the vessel wall motion state, the combined IVUS images correspond to a same vessel wall motion state, thereby reducing artifacts or even eliminate artifacts in the resulting combined IVUS image, i.e. in the resulting longitudinal IVUS view of the vessel, which might generally be caused by combining IVUS image corresponding to different vessel wall motion states. Moreover, since it is not required to use an ECG for selecting the IVUS images to be combined, the handling of the image generation procedure can be simplified, because it is not required to equip a patient with ECG electrodes.

The ultrasound images providing unit can be a storing unit in which the IVUS images of the vessel are stored and from which the IVUS images can be retrieved for providing the same. Moreover, the ultrasound images providing unit can be a receiving unit for receiving the IVUS images from an intravascular ultrasound images generation unit and for providing the received IVUS images. Furthermore, the ultrasound images providing unit can be adapted to receive ultrasound signals from an ultrasound signals acquisition unit, to determine the IVUS images based on the received ultrasound signals and to provide the generated IVUS images. The ultrasound images providing unit can also include the ultrasound signals acquisition unit. The provided IVUS images are preferentially cross-sectional IVUS images of the vessel at the respective acquisition location.

The acquisition locations providing unit can be a storing unit in which the different acquisition locations are stored and from which the different acquisition locations are retrieved for providing the same. The acquisition locations providing unit can also be a receiving unit for receiving the different acquisition locations from, for instance, an acquisition location measuring unit and for providing the received acquisition locations. Moreover, the acquisition locations providing unit can also be the acquisition locations measuring unit itself. The acquisition locations providing unit can be adapted to provide absolute locations or relative locations as the acquisition locations, wherein a relative location is preferentially a location along the length of the vessel relative to the previous location for which an ultrasound image has been provided, which fulfills the above described pressure-related condition and optionally, i.e. depending on the respective embodiment, one or more of the below described temporal and spatial conditions. The preferred relative location can also be defined as a location along the length of the vessel relative to a previous location for which an ultrasound image has been provided, which is used for the combination with the other provided ultrasound images.

Also, the pressure values providing unit can be a storing unit, wherein in this case the storing unit is adapted to store intravascular pressure values and to provide the stored intravascular pressure values. The pressure values providing unit can also be a receiving unit for receiving the intravascular pressure values from a pressure values measuring unit and for providing the received intravascular pressure values. Moreover, the pressure values providing unit can also be the pressure values measuring unit.

It is preferred that the ultrasound images combining unit is adapted to combine provided IVUS images which have been determined based on IVUS signals acquired at acquisition times which differ by less than a predefined upper temporal deviation threshold. This can ensure that relatively slow variations in movements of the vessel wall have no or only limited effect on the pressure values. The pressure values can therefore be a further improved measure for the vessel wall motion state, which can lead to a further improved quality of the combined ultrasound image.

Moreover, it is preferred that the ultrasound images combining unit is adapted to combine provided intravascular ultrasound images which have been determined based on IVUS signals acquired at acquisition times which differ by more than a predefined lower temporal deviation threshold. In particular, the ultrasound image generation system further comprises a cardiac period value providing unit for providing a cardiac period value being indicative of a temporal length of a cardiac cycle, wherein the lower temporal deviation threshold is predefined such that it is equal to or larger than the temporal length of the cardiac cycle as indicated by the provided cardiac period value. The lower temporal deviation threshold can also be larger than the temporal length of several cardiac cycles. Since the vessel wall motion is mainly caused by the cardiac motion, by combining IVUS images, which have been acquired at acquisition times which differ by more than a temporal length of a cardiac cycle, it can be ensured that the combined IVUS images correspond to different vessel wall motion periods and not to a same vessel wall motion period, thereby ensuring that the combined IVUS images correspond to a same vessel wall motion state in different vessel wall motion periods. This can lead to a further improved image quality of the resulting combined IVUS image, i.e. of the longitudinal view of the vessel.

The cardiac period value is preferentially predefined and stored in the cardiac period value providing unit, wherein the cardiac period value providing unit is adapted to provide the stored cardiac period value. Thus, preferentially it is not necessary to measure, for instance, an electrocardiogram while acquiring IVUS signals for providing the cardiac period value. The cardiac period value can be based on an estimation and/or on previous measurements of the temporal length of the cardiac cycle of the specific patient or of a group of patients being similar to the specific patient with respect to predefined criteria like age, gender, et cetera.

It is also preferred that the ultrasound images combining unit is adapted to combine provided IVUS images which have been determined based on IVUS signals acquired at acquisition locations which differ by less than a predefined spatial deviation threshold. This can significantly reduce the likelihood that a pressure change is caused to a large extent by changing vessel morphology along the length of the vessel and not by vessel wall motion, thereby further increasing the likelihood that IVUS images corresponding to a same vessel wall motion state are combined, which can lead to a further increased image quality of the of the longitudinal view of the vessel.

The ultrasound images combining unit can be adapted to provide a morphology of the vessel and to modify the predefined spatial deviation depending on the provided morphology. In particular, the ultrasound images combining unit can be adapted to determine the gradient of the open cross-section of the vessel along the length of the vessel, wherein the spatial deviation threshold can be modified, i.e. determined, based on the gradient at the current location within the vessel of, for instance, an ultrasound signals acquisition unit. For this determination and thereby modification of the spatial deviation threshold assignments between gradients and spatial deviation thresholds or between gradients and modifications of the spatial deviation thresholds can be used, which might be defined during a calibration procedure and/or by simulation. The morphology of the vessel might be known already from previous measurements of the vessel with IVUS or another imaging modality, wherein the ultrasound images combining unit can be adapted to provide this already known morphology.

It is preferred that the ultrasound images combining unit is adapted to combine temporally adjacent IVUS images. Thus, the ultrasound images combining unit is preferentially adapted to combine the IVUS images pairwisely. The ultrasound images combining unit can therefore be adapted to combine, for instance, a) a temporally first IVUS image with a temporally second IVUS image, wherein for both IVUS images the above described pressure-related condition and optionally, i.e. depending on the respective embodiment, one or more of the further above described conditions are fulfilled, b) the temporally second IVUS image with a temporally third IVUS image, wherein for both IVUS images the above described pressure-related condition and optionally one or more of the further above described conditions are fulfilled, c) the temporally third IVUS image with a temporally fourth IVUS image, wherein for both IVUS images the above described pressure-related condition and optionally one or more of the further above described conditions are fulfilled, et cetera, in order to generate the combined IVUS image showing the length of the vessel covering the different acquisition locations. In other words, preferentially the expression "*temporally adjacent*" refers just to the set of IVUS images which fulfill the one or more conditions which should be fulfilled in the respective embodiment, wherein the temporally first IVUS image of this set is combined with the temporally second IVUS image of this set, the temporally second IVUS image of this set is combined with the temporally third IVUS image of this set, and so on. The process of combining two IVUS images is preferentially a stitching process, wherein the two IVUS images are just stitched together. In an embodiment, the ultrasound images combining unit can also be adapted to register the two IVUS images before being combined and to stitch the registered IVUS images together. The registration is preferentially a registration, which is based on features detectable in the IVUS images to be combined.

In an embodiment the ultrasound image generation system further comprises a controller for controlling an ultrasound signals acquisition unit for acquiring the IVUS signals, while the ultrasound signals acquisition unit is moved within and to different locations along the length of the vessel by using a moving unit, wherein the pressure values providing unit is adapted to provide pressure values being indicative of the current pressure within the vessel while the ultrasound signals acquisition unit is moved, wherein the controller is adapted to control the ultrasound signals acquisition unit such that, for determining an IVUS image, IVUS signals are acquired, if a difference between a current pressure value and the reference pressure value being a pressure value provided for a last acquisition time at which IVUS signals for determining a previous IVUS image have been acquired, is smaller than the predefined pressure deviation threshold, wherein the ultrasound images providing unit is adapted to provide the IVUS images by determining them based on the acquired IVUS signals. Thus, the controller can be adapted to control the ultrasound signals acquisition unit such that the acquired IVUS signals and hence the provided IVUS images already fulfill the condition that they should correspond to similar pressure values. The controller can be further adapted to control the ultrasound signals acquisition unit such that the ultrasound signals are only acquired, if also one or more of the further, above mentioned conditions are fulfilled. Thus, the controller can be adapted to control the ultrasound signals acquisition unit such that the IVUS signals are acquired only, if the currently measured pressure is similar to a pressure corresponding to the IVUS signals acquired at the last acquisition location and optionally a) if the current time differs by less than the predefined upper temporal deviation threshold from the last acquisition time at which the IVUS signals have been acquired and/or b) if the current time differs by more than a predefined lower temporal deviation threshold from the last acquisition time at which an IVUS signal has been acquired and/or c) if the current location of the ultrasound signals acquisition unit differs by less than the predefined spatial deviation threshold from the last acquisition location at which the IVUS signals have been acquired. The pressure measurement and optionally also the time and location measurements can therefore be used for controlling the acquisition of the IVUS signals and hence the determination and provision of the IVUS images. However, it is also possible that the acquisition of the IVUS signals is not controlled in such a way and that also IVUS signals are acquired and corresponding IVUS images are determined, which do not fulfill the above mentioned conditions. A selection of the IVUS images to be combined such that the above mentioned conditions are fulfilled is then carried out by the ultrasound images combining unit. Thus, instead of a gated acquisition, it is also possible to use a retrospective gating.

The reference pressure value can be a pressure value that has been measured at an acquisition time at which IVUS signals have been acquired. For instance, it can be the pressure value, which has been measured at the earliest acquisition time of the acquisition times corresponding to IVUS measurements performed during a pullback movement of the IVUS acquisition unit in the vessel. The reference pressure value can also be adapted during the IVUS acquisition. For example, the reference pressure value can be the pressure value that has been measured while the IVUS signals for the last IVUS image, which fulfils the above described pressure condition and preferentially the above described time and location conditions, have been acquired.

The controller and the ultrasound images providing unit are preferentially integrated such that the controller is also adapted to determine the IVUS images based on the acquired IVUS signals. However, the controller and the ultrasound images providing unit can also be different units. Moreover, in a preferred embodiment the controller is adapted to control the ultrasound signals acquisition unit and also the moving unit such that the ultrasound signals are acquired at acquisition locations having a predetermined spatial resolution. In particular, the controller can be adapted to control a) the moving unit such that, after IVUS signals have been acquired at an acquisition location, the ultrasound signals acquisition unit is moved to a next acquisition location which corresponds to the predetermined spatial resolution, and b) the ultrasound signals acquisition unit such that IVUS signals are acquired at the next acquisition location if a current pressure differs by less than the predefined deviation threshold from the reference pressure value being a pressure value measured when acquiring the IVUS signals at the previous acquisition location and optionally if one or both of the above described temporal conditions are fulfilled. By determining a combined ultrasound image, which has a predetermined, especially a constant, spatial resolution, the image quality of the combined ultrasound image can be further improved.

In an embodiment the ultrasound images combining unit is adapted to generate several combined ultrasound images for different reference pressure values, wherein for generating a respective combined ultrasound image provided IVUS images are combined, which have been determined based on IVUS signals acquired at acquisition times at which the pressure values differ by less than a predefined deviation threshold from the respective reference pressure value, depending on the acquisition locations. Thus, for different vessel wall motion states different combined ultrasound images showing the length of the vessel can be generated, wherein these several combined ultrasound images form a temporal combined ultrasound image showing the vessel wall motion.

The pressure values providing unit is preferentially adapted to provide the intravascular pressure values such that they are indicative of the pressure within the vessel at the acquisition locations. Thus, preferentially the pressure values are directly measured at the acquisition locations. This can further increase the quality of the pressure values with respect to their suitability for choosing which IVUS images should be combined for generating the combined ultrasound image showing the length of the vessel. This can lead to a further increased image quality of the combined ultrasound image.

Moreover, preferentially the ultrasound images providing unit and the pressure values providing unit are integrated in a single unit. For instance, the ultrasound images providing unit can include the ultrasound signals acquisition unit which might comprise a catheter with an ultrasound transducer for acquiring the IVUS signals, wherein the ultrasound transducer can also be used for measuring the pressure and hence for generating the pressure values or wherein an additional sensor can be used for measuring the pressure, wherein this additional sensor might also be attached to the catheter. By using a same unit for providing the ultrasound images and for providing the pressure values, the handling of the ultrasound image generation system can be simplified for a user.

In a further aspect of the present invention, an ultrasound image generation method for generating an intravascular ultrasound image is presented, wherein the ultrasound image generation method comprises:
- providing intravascular ultrasound images of a vessel, which have been determined based on intravascular ultrasound signals acquired at different acquisition times and at different acquisition locations along the length of the vessel by an ultrasound images providing unit,
- providing the different acquisition locations, at which the intravascular ultrasound signals have been acquired, by an acquisition locations providing unit,
- providing intravascular pressure values being indicative of the pressure within the vessel at the acquisition times by a pressure values providing unit,
- generating a combined ultrasound image by combining provided intravascular ultrasound images, which have been determined based on intravascular ultrasound signals acquired at acquisition times at which the pressure values differ by less than a predefined deviation threshold from a reference pressure value, depending on the acquisition locations by an ultrasound images combining unit.

In another aspect of the present invention a computer program for generating an intravascular ultrasound image is presented, wherein the computer program comprising program code means for causing an ultrasound image generation system as defined in claim 1 to carry out the steps of the ultrasound image generation method as defined in claim 14, when the computer program is run on a computer controlling the ultrasound image generation system.

It shall be understood that the ultrasound image generation system of claim 1, the ultrasound image generation method of claim 14, and the computer program of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of an ultrasound image generation system for generating an intravascular ultrasound image,
Fig. 2 shows schematically and exemplarily a distal end of a catheter with an intravascular ultrasound transducer of the ultrasound image generation system,
Fig. 3 shows an example of an intravascular pressure measurement, and
Fig. 4 shows a flowchart exemplarily illustrating an embodiment of an ultrasound image generation method for generating an intravascular ultrasound image.

### DETAILED DESCRIPTION OF EMBODIMENTS

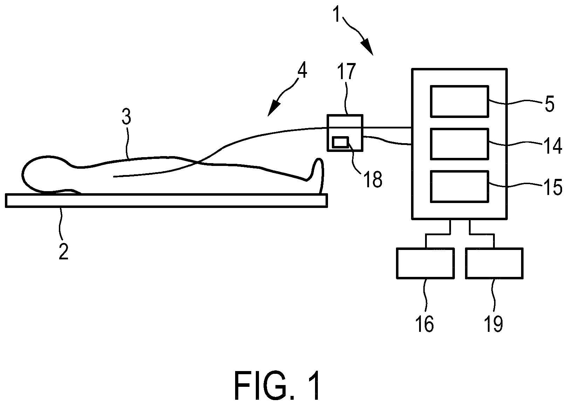

Fig. 1 shows schematically and exemplarily an embodiment of an ultrasound image generation system for generating an intravascular ultrasound image. The ultrasound image generation system 1 comprises a catheter 4 being adapted to be introduced into a vessel of a person 3 lying on a support means 2 like a patient table. After the catheter 4 has been introduced into the vessel of the person 3 and moved to a desired location within the vessel, the catheter 4 can be pulled back manually or by using a moving unit 17. The moving unit 17 is an automatic pullback device that pulls the catheter 4 back and out of the vessel within the person 3. The moving unit 17 can also be adapted to move the catheter 4 in a forward direction within the vessel in the person 3, i.e. to move the catheter 4 further into the person 3. However, also the forward movement can be carried out manually.

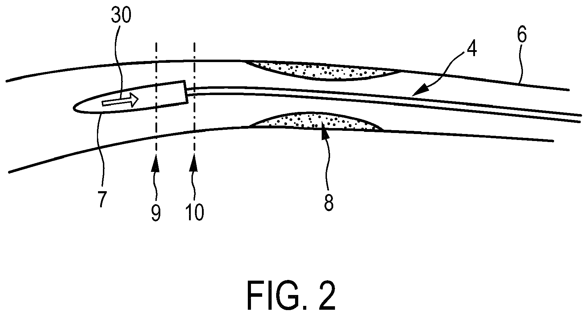

The distal part of the catheter 4 is schematically and exemplarily illustrated in Fig. 2. The catheter 4 comprises an IVUS transducer 7 that is movable in a pullback direction 30 via the catheter 4 by using the moving unit 17. Fig. 2 also schematically and exemplarily shows the vessel 6 with a stenosis 8. The IVUS transducer 7 is adapted to send and receive ultrasound waves for generating ultrasound signals that can be used for determining a cross-sectional IVUS image at the respective location of the IVUS transducer 7. The acquired ultrasound signals are then sent to a controller 5 via the catheter 4, wherein the controller 5 is adapted to generate cross-sectional IVUS images at different locations along the length of the vessel 6, while the IVUS transducer 7 is moved in the pullback direction 30. The catheter 4 with the IVUS transducer 7 and the controller 5 provide therefore IVUS images of the vessel 6, which have been determined based on IVUS signals acquired at different locations along the length of the vessel 6 and at different acquisition times. The catheter 4 with the IVUS transducer 7 and the controller 5 can therefore be regarded as being components of an ultrasound images providing unit.

In this embodiment, the IVUS transducer 7 is also adapted to measure the pressure within the vessel at the respective location of the IVUS transducer 7, wherein the measured pressure is sent to the controller 5 via the catheter 4. The catheter 4 with the IVUS transducer 7 therefore also provides intravascular pressure values being indicative of the pressure within the vessel. The catheter 4 with the IVUS transducer 7 can therefore also be regarded as being a pressure values providing unit.

Thus, in this embodiment a same device, i.e. the catheter 4 with the IVUS transducer 7, is used for providing the cross-sectional IVUS images and for providing the pressure values, wherein the pressure values can be directly measured at the locations along the length of the vessel 6 at which also the IVUS signals are acquired. In another embodiment it is also possible that an additional sensor, which is preferentially also integrated with the catheter 4, is used for measuring the pressure close to the location of the IVUS transducer 7.

The ultrasound image generation system further comprises a locations measuring sensor 18 arranged at the moving unit 17, wherein the locations measuring sensor 18 is adapted to measure at the moving unit 17 how far the IVUS transducer 7 has been moved within the vessel 6, wherein this information is used for determining the respective location of the IVUS transducer 7 along the length of the vessel 6. The determined location is preferentially a relative location, i.e. a location relative to a last acquisition location at which IVUS signals have been acquired and for which a corresponding IVUS image has been determined based on these IVUS signals. The locations measuring sensor 18 can also be adapted to measure the locations relative to another location along the length of the vessel, i.e., for instance, relative to the furthermost location of the IVUS transducer 7 to which the IVUS transducer 7 was moved forward into the vessel 6, before the pullback procedure started. Since the locations measuring sensor 18 is adapted to measure the locations of the IVUS transducer 7 within the vessel 6 and since these measured locations also include the locations at which the IVUS signals are acquired, which are used for determining the cross-sectional IVUS images, the locations measuring unit 18 can be regarded as being an acquisition locations providing unit for providing different acquisition locations at which the IVUS signals, which are used for determining the cross-sectional IVUS images, are acquired. The locations measuring sensor 18 can be, for instance, an incremental encoder. However, also other means can be used for providing the acquisition locations. For instance, the acquisition locations providing unit can be adapted to provide the acquisition locations based on angiography, i.e. based on an image of the vessel.

The ultrasound image generation system 1 further comprises a cardiac period value providing unit 15 for providing a cardiac period value being indicative of a temporal length of a cardiac cycle. In this embodiment, the cardiac period value providing unit 15 is a storing unit in which a cardiac period value is stored being indicative of a typical temporal length of the cardiac cycle of the person 3 lying on the table 2. For instance, the heart rate, which is indicative of the temporal length of the cardiac cycle, can be stored in the cardiac period value providing unit 15. In another embodiment, the cardiac period value providing unit 15 may be a measuring unit like an electrocardiograph for measuring the temporal length of the cardiac cycle of the person 3. However, in a further embodiment the cardiac period value providing unit 15 can also not be present. In fact, in another embodiment the ultrasound image generation system 1 does not necessarily need the cardiac period value providing unit 15.

While the moving unit 17 pulls back the catheter 4 with the IVUS transducer 7, the location of the IVUS transducer 7 and the pressure at the respective location are continuously measured. Also, the time is continuously measured by, for instance, the controller 5 or by another component which is able to measure the time. The controller 5 is adapted to control the IVUS transducer 7 such that it acquires IVUS signals only, if the following conditions are fulfilled: a) the difference between a current pressure value and a reference pressure value being a pressure value provided for a last acquisition time, at which IVUS signals for determining a previous IVUS image have been acquired, is smaller than a predefined pressure deviation threshold, b) a difference between a current time and a previous acquisition time, at which IVUS signals have been previously acquired for determining an IVUS image, is smaller than a predefined upper temporal deviation threshold, c) this temporal difference is larger than a predefined lower temporal deviation threshold, and d) a difference between the current location and a previous acquisition location, at which IVUS signals have been acquired, which have been used for determining a previous cross-sectional IVUS image, is smaller than a predefined spatial deviation threshold. Thus, the controller 5 is adapted to continuously check whether these conditions are fulfilled, wherein, if this is the case, next IVUS signals are acquired and used for determining a next cross-sectional IVUS image.

The ultrasound image generation system further 1 comprises an ultrasound images combining unit 14 for generating a combined ultrasound image by combining the provided IVUS images depending on the respective acquisition locations. Preferentially, the ultrasound images combining unit 14 is adapted to stitch together temporally adjacent IVUS images for generating the combined ultrasound image which is a longitudinal ultrasound image showing the vessel 6 along its length. In particular, the cross-sectional IVUS images, which correspond to different acquisition locations along the length of the vessel 6, are pairwisely stitched together, in order to generate the combined ultrasound image. For this combination or stitching process known registration-based algorithms can be used like the algorithm disclosed in, for instance, the article "Rigid and elastic registration for coronary artery IVUS images" by Z. Sun et al., Technological and Health Care, volume 2, pages 455 to 463 (2016) which is herewith incorporated by reference. The stitching process can also be carried out without registering the IVUS images to be combined, i.e. the respective two IVUS images can be just stitched together. The stitching process could also be regarded as being a stacking process wherein the two IVUS images are stacked together.

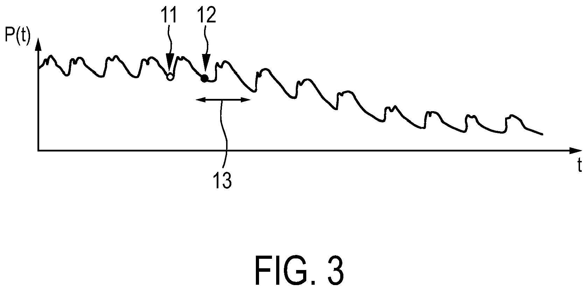

In Fig. 2 acquisition locations, at which the above described conditions are fulfilled and IVUS signals, which are used for determining corresponding cross-sectional IVUS images, are indicated by lines 9 and 10. Fig. 3 schematically and exemplarily illustrates a pressure measurement over time and hence over the locations along the length of the vessel 6, because the IVUS transducer 7 is moved in the pullback direction 30 out of the vessel 6. Acquisition times, which correspond to the acquisition locations 9, 10, are indicated in Fig. 3 by the dots 11, 12, respectively.

The predefined upper temporal deviation threshold, the predefined spatial deviation threshold and the predefined lower temporal deviation threshold define a spatio-temporal correlation window, i.e. a window having temporal and spatial aspects or, in other words, a temporal window and a spatial window. In Fig. 3, reference sign "13" schematically and exemplarily indicates the temporal aspect of the spatio-temporal correlation window or, in other words, the temporal window. If the difference between a current pressure value and the reference pressure value being the pressure value provided for a last acquisition time, at which IVUS signals for determining a previous IVUS image have been acquired, is smaller than the predefined pressure deviation threshold and if the current location of the IVUS transducer and the current time are within the spatio-temporal correlation window, next IVUS signals are acquired and used for determining a next cross-sectional IVUS image.

The controller 5 can be further adapted to control the IVUS transducer 7 and the moving unit 17 such that the ultrasound signals are acquired at acquisition locations 9, 10 having a constant spatial resolution, wherein this spatial resolution might be selectable by a user via an input unit 16 like a keyboard, a computer mouse, a touch pad, et cetera. In particular, the controller 5 is adapted to control a) the moving unit 17 such that, after IVUS signals have been acquired at an acquisition location 9, the IVUS transducer 7 is moved to a next acquisition location 10 which corresponds to a desired constant spatial resolution, and b) the IVUS transducer 7 such that ultrasound signals are acquired at this next acquisition location 10, if the above described conditions with respect to pressure and time are fulfilled. After the IVUS signals have been acquired at this next acquisition location 10, the moving unit 17 moves the IVUS transducer 7 to a further acquisition location which still corresponds to the desired constant spatial resolution, wherein also at this further acquisition location IVUS signals are acquired, if the above described conditions with respect to time and pressure are fulfilled. This procedure can proceed, until a desired number of cross-sectional ultrasound images have been acquired. The constant spatial resolution corresponds preferentially to a distance between neighboring acquisition locations, which is smaller than the predefined spatial deviation threshold.

The acquisition of the ultrasound signals, only if the above described conditions are fulfilled, is a gated acquisition. Instead of or in addition to gating already during the acquisition, in an embodiment the ultrasound images combining unit can also be adapted to provide a retrospective gating. For instance, while the IVUS transducer 7 is pulled back out of the vessel 6, IVUS signals can be acquired, corresponding cross-sectional IVUS images can be determined based on these acquired IVUS signals, the locations of the IVUS transducer 7, the pressure values at these locations of the IVUS transducer 7 and the time can be measured, and this ungated information can be provided to the ultrasound images combining unit, wherein the ultrasound images combining unit can determine which of the cross-sectional IVUS images fulfill the above described conditions with respect to the pressure values, acquisition times and acquisition locations and then only combine these cross-sectional IVUS images which fulfill these conditions.

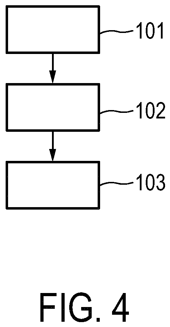

In the following, an embodiment of an ultrasound image generation method for generating an IVUS image will exemplarily be described with reference to a flowchart shown in Fig. 4.

In step 101 the moving unit 17 pulls the catheter 4 with the IVUS transducer 7 in the pullback direction 30 within the vessel 6, while the locations measuring sensor 18 measures the locations of the IVUS transducer 7, the IVUS transducer 7 is used for measuring the pressure at the respective location and the time is measured. Moreover, still in step 101, if the above described conditions with respect to location, time and pressure are fulfilled, IVUS signals are acquired at the respective location, i.e. at the respective acquisition location, and an IVUS image is determined based on the IVUS signals for the respective acquisition location.

In step 102, the ultrasound images combining unit 14 combines the IVUS images, which have been acquired at the different acquisition locations, depending on the different acquisition locations, in order to generate a combined ultrasound image showing the vessel 6 along its length.

Since the acquisition of the IVUS signals and the corresponding determination of the IVUS images in step 101 is gated with respect to the above described conditions, the ultrasound images combining unit 14 only combines IVUS images fulfilling these conditions. If in another embodiment the acquisition of the IVUS signals and the corresponding determination of the IVUS images were not gated with respect to the above described conditions for the pressure values, the locations and the times, in step 102 the ultrasound images combining unit 14 would check for which IVUS images these conditions are fulfilled and then only combine these IVUS images.

In step 103, the combined ultrasound image is shown on a display 19.

The ultrasound image generation system and method are particularly useful for diagnosing coronary artery disease. For instance, they are very useful for evaluating coronary plaque burden or for characterizing a stenosis. The ultrasound image generation system and method can also be very helpful as an imaging support in the context of stenting.

The IVUS images, which are preferentially cross-sectional IVUS images and which are recorded during the pullback procedure, are joined together such that temporally adjacent IVUS images, which should be combined, are aligned. The resulting combined ultrasound image is a longitudinal ultrasound view, i.e. a view along the vessel.

If the IVUS images, which are combined, were not gated by using the above described conditions, the combined ultrasound image would show artifacts due to movements of the vessel wall, which are caused by the beating heart and intravascular pressure changes. These artifacts would be, for instance, sawtooth-like image artifacts. In order to avoid these artifacts, the above described ultrasound image generation system and method use the pressure, location and time based conditions for the gating. However, the ultrasound image generation system and method do not use an electrocardiography gating for the following reasons.

An electrocardiography gating would require a recording of electrocardiography signals, which adds effort to the clinical workflow as well as inconvenience for the person to be examined during the invasive procedure. Furthermore, the electrocardiography recording can be seen as an error source linked to a human operator, wherein corresponding errors may relate to the electrode placement, the contact impedance, et cetera. This error source linked to the human operator can compromise the longitudinal representation of the finally combined ultrasound image. Moreover, poor quality longitudinal views of the vessel originating from inappropriate electrocardiography recordings can generally only be noticed retrospectively, when options for corrections are limited. In addition, electrocardiography only provides a general electrophysiological signal and is not linked to specific locations along or at the respective vessel that is preferentially a coronary. One example for a non-systematic error source is the delay interval between the QRS complex of the electrocardiography signal and the mechanical contraction of the heart, wherein this delay interval may vary over time. Using electrocardiography as a proxy for, for instance, coronary motion is therefore often not very accurate, particularly in persons suffering from cardiac dysfunction. The ultrasound image generation system and method described above with reference to Figs. 1 to 4 exploit therefore a surrogate measure, i.e. the provided pressure values which are closer to the site-of-interest and do not require external electrocardiography measurements, i.e. do not require the aforementioned additional effort.

The ultrasound image generation system and method are adapted to gate IVUS recordings by using intravascular blood pressure measurements in the respective vessel that is preferentially a coronary. For measuring the intravascular blood pressure a capacitive sensor in the IVUS transducer 7 itself might be used. However, it is also possible to use one or several additional sensors attached to, for instance, the catheter 4, which are adapted to only measure the intravascular blood pressure. In addition to this pressure measurement, the ultrasound image generation system and method is preferentially adapted to require a feedback of time and relative catheter location to define the spatio-temporal correlation window for the pressure signal, wherein this spatio-temporal correlation window is defined by the above described upper and lower temporal deviation thresholds and the spatial deviation threshold.

The information with respect to the catheter location can be obtained by one or several sensors at the automatic pullback device. In particular, the above described locations measuring sensor 18 can be used for providing the required information regarding the catheter location, especially regarding the location of the IVUS transducer 7. If the moving unit 17, which might be the automatic pullback device, or another unit like the controller 5 knows how much of the catheter 4 has been pulled back at a certain time, an additional sensor for determining the respective location might not be required and the location might directly be provided by, for instance, the moving unit or the controller 5. In this case, the moving unit or the controller, respectively, might be regarded as being an acquisition locations providing unit for providing the acquisition locations at which the IVUS signals have been acquired. The ultrasound image generation system and method described above with reference to Figs. 1 to 4 join and correlate the time, location and pressure information to obtain information about when to acquire the desired IVUS signals and hence to determine the desired IVUS images and to monitor whether the current pressure is still within the spatio-temporal bounds which allow for a direct comparison of pressure values recorded by, for instance, a capacitive sensor in the IVUS transducer 7.

Given a pressure measurement at a certain location along the length of the vessel and at a certain time, it is assumed that the pressure signal will roughly return to the pressure measurement, which might be regarded as defining a reference pressure value, in subsequent cardiac cycles. IVUS images, which are preferentially cross-sectional images, recorded in these corresponding points are used for stitching together a longitudinal view of the vessel. The challenge with this assumption is that the pressure signal is non-stationary. This means that it does not only depend on the heartbeat, i.e. on the contraction and the expansion of the cardiac system, but also on the morphology of the vessel, particularly the coronary, at the current catheter position as well as the long-term hemodynamics of the vascular system. The latter can be influenced by many external factors such as arousal or relaxation. Since the pressure signal can be subject to these misleading and uninformative variations, the above described conditions are preferentially used, which relate to the times and locations and which might also be regarded as defining a spatio-temporal correlation window. This window can make sure that the catheter, i.e. the IVUS transducer 7 at the tip of the catheter, does not significantly move along the vessel centerline such that a pressure change might not have been affected to a large extent by changing vessel morphology. An extreme case of such a pressure change caused by stenosis 8 is illustrated in Fig. 2.

As is shown in Fig. 2 in combination with Fig. 3, while the IVUS transducer 7 is pulled back along the vessel 6, the pressure signal p is continuously recorded, wherein this pressure signal p depends on the time t and hence, since the catheter 4 with the IVUS transducer 7 is moved in the pullback direction 30, on the respective location x along the length of the vessel 6. The pressure signal p exhibits a superposition of pressure variations over time, i.e., for instance, across a cardiac cycle and due to long-term hemodynamics. Also, the pressure drop along the vessel caused by the stenosis 8 is illustrated in Fig. 3.

The spatial variation of the pressure p follows a certain decay pattern that is, however, locally more or less stable. A pressure recording at a first time 11 and a corresponding first location 9 has a certain spatio-temporal neighborhood in which the pressure will still return to about the same level again, which in Figs. 2 and 3 is denoted by reference numbers 10 and 12. Cross-sectional views, i.e. IVUS images, which are acquired at these two points, can be selected as adjacent cross-sectional views to be combined to a longitudinal view. The correlation window moves with the pullback and may change in length depending on the anatomy. In particular, the ultrasound images combining unit 14 can be adapted to provide a morphology of the vessel and to modify the predefined spatial deviation threshold and hence the length of the spatial aspect of the correlation window depending on the provided morphology. For example, the ultrasound images combining unit 14 can be adapted to determine the gradient of the open cross-section, i.e. of the inner cross-section or lumen, of the vessel along the length of the vessel, wherein the spatial deviation threshold can be modified, i.e. determined, based on the gradient at the current location within the vessel of the IVUS transducer 7. For this determination and thereby modification of the spatial deviation threshold assignments between gradients and spatial deviation thresholds or between gradients and modifications of the spatial deviation thresholds can be used, which might be defined during a calibration procedure and/or by simulation. The morphology of the vessel might be known already from previous measurements of the vessel with IVUS or another imaging modality, wherein the ultrasound images combining unit 14 can be adapted to provide this already known morphology. However, if a retrospective gating is used, also the acquired ungated IVUS images can be used for determining the morphology, i.e., for instance, the ungated IVUS can be stitched together and the vessel can be segmented in the resulting ungated IVUS overall image for determining the morphology.

Temporally the spatio-temporal correlation window is preferentially defined such that the measured pressure value falls within a certain time interval since the pressure value linked to a last cross-sectional IVUS image was recorded. If this is the case, long-term, i.e. slowly varying, hemodynamics have only limited effect on the pressure signal, and only the much faster heartbeat which is subject to the gating process might have an effect.

By using the above described conditions, the ultrasound image generation system and method ensure that a comparison between a reference pressure value of a last cross-sectional IVUS image and newly acquired pressure values is only valid within the correlation window. Thus, it can be ensured that a most similar, i.e. in an optimal case equal, pressure value and hence location along the length of the vessel is selected for acquiring a next cross-sectional IVUS image, which is still within the window. The reference pressure value is preferentially always updated to that of the last acquisition location, i.e. of the last chosen cross-sectional IVUS image, wherein this allows for variations of the baseline signal that are slower than the heartbeat.

The above described moving unit 17 can be adapted to provide a continuous, automatic pullback, wherein the correlation window internally defines the valid acquisition context. During the pullback, the pressure signal is recorded, i.e. the pressure values are measured by using the pressure measuring sensor 18, and a new acquisition is triggered when the current pressure signal is similar to the reference of the previously acquired cross-sectional IVUS image and the recording is valid according to the correlation window. If a heartbeat rate of about 1 to 2 Hz and a pullback speed between 0.5 and 1 mm/s were assumed, a heartbeat would always contain a certain distance that might be, with a heartbeat rate of 1 Hz and a pullback speed of 1 mm/s, 1 mm. This distance is then the lower bound of the spatial resolution along the centerline. The temporal aspect of the correlation window, i.e. here the lower temporal deviation threshold, always exceeds the duration of at least one heartbeat, but preferentially includes several heartbeats, i.e. exceeds the duration of several heartbeats. In this example the spatial resolution is only approximate, but not ensured, because it depends on the heart rate and exact pressure variations. Only images which are considered as qualified by the gating process are acquired or, if they have been acquired, used for generating the combined ultrasound image.

In a further example, a certain spatial resolution can be ensured which might be constant. In particular, a clinician can define a desired spatial resolution along the vessel centerline by using, for instance, the input unit 16. The pullback device 17, i.e. the moving unit, can then be asked to proceed in a varying speed or discrete spatial steps with the pullback, wherein for this procedure a backward feedback can be used. In the backward feedback the ultrasound imaging process can signal whenever an ultrasound acquisition could be made that has the appropriate pressure level and is still within the bounds of the correlation window constraint, wherein in this case clearance to the pullback device is given for proceeding to the next sample point in accordance with the desired spatial resolution. Thus, the controller can control the pullback device such that the IVUS transducer 7 is moved to the next sample point, when an IVUS image has been acquired in accordance with the above described pressure, time and location conditions. That means the pullback velocity can be adapted online depending on the current heart rate to achieve an approximately equidistant sampling of cross-sectional IVUS images along the vessel. Also in this example, in contrast to other measurements like electrocardiography measurements, the described pressure measurements provide a more direct feedback, i.e. the feedback has much less latency and is more closely linked to the site of interest. Therefore, the intravascular pressure measurements are very suited for this kind of "*control loop*" to the pullback device.

As explained above, the ultrasound image generation system and method can also be configured to be used retrospectively. Cross-sectional IVUS images and pressure values can be acquired simultaneously at different locations along the length of the vessel without gating, wherein during offline processing afterwards the ultrasound images can be sorted according to the vessel wall motion state, which might correspond to a pressure-defined heart phase, in order to provide different longitudinal views for different vessel wall motion states or different heart phases, respectively. Also, this retrospective intravascular pressure based gating does still not require, for instance, electrocardiography recordings and does also not only rely on retrospectively computed, mutual image similarity measures.

The ultrasound image generation system and method can be used for, for instance, plaque burden or lesion monitoring for acute coronary syndromes (ACS) or stable angina. The ultrasound image generation system and method can also be adapted to be used in the context of stenting and/or follow-up imaging. In these applications, the described ultrasound image generation system and method can increase the value of longitudinal overviews across entire vessel segments as well as improve inspection of the vessel or treatment characteristics that stretch along the vessel. The ultrasound image generation system and method can allow for a more realistic, equidistant and controllable sampling along the vessel.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like providing the IVUS images, providing the acquisition locations, providing the pressure values, generating the combined ultrasound image, et cetera performed by one or several units or devices can be performed by any other number of units or devices. These procedures and/or the control of the ultrasound image generation system in accordance with the ultrasound image generation method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to the field of IVUS imaging. IVUS images of a vessel are provided, which have been determined based on IVUS signals acquired at different acquisition locations along the length of the vessel and at different acquisition times. Moreover, pressure values are provided, which are indicative of the pressure within the vessel at the acquisition times, wherein a combined IVUS image is generated by combining provided IVUS images that have been determined based on IVUS signals acquired at acquisition times at which the pressure values differ by less than a predefined deviation threshold from a reference pressure value. This use of the pressure within the vessel as a selection criterion for combining IVUS images can ensure that only IVUS images are combined, which correspond to a same vessel wall motion state, thereby reducing artifacts in the combined IVUS image.

## Claims

1. An ultrasound image generation system for generating an intravascular ultrasound image, wherein the ultrasound image generation system (1) comprises:
- an ultrasound images providing unit (4, 5) for providing intravascular ultrasound images of a vessel (6), which have been determined based on intravascular ultrasound signals acquired at different acquisition locations (9, 10) along the length of the vessel (6) and at different acquisition times,
- an acquisition locations providing unit (18) for providing the different acquisition locations at which the intravascular ultrasound signals have been acquired,
- a pressure values providing unit (4) for providing intravascular pressure values being indicative of the pressure within the vessel (6) at the acquisition times (11, 12),
- an ultrasound images combining unit (14) for generating a combined ultrasound image by combining provided intravascular ultrasound images, which have been determined based on intravascular ultrasound signals acquired at acquisition times (11, 12) at which the pressure values differ by less than a predefined deviation threshold from a reference pressure value, depending on the acquisition locations (9, 10).

2. The ultrasound image generation system as defined in claim 1, wherein the ultrasound images combining unit (14) is adapted to combine provided intravascular ultrasound images which have been determined based on intravascular ultrasound signals acquired at acquisition times (11, 12) which differ by less than a predefined upper temporal deviation threshold.

3. The ultrasound image generation system as defined in claim 1, wherein the ultrasound images combining unit (14) is adapted to combine provided intravascular ultrasound images which have been determined based on intravascular ultrasound signals acquired at acquisition times (11, 12) which differ by more than a predefined lower temporal deviation threshold.

4. The ultrasound image generation system as defined in claim 3, wherein the ultrasound image generation system (1) further comprises a cardiac period value providing unit (15) for providing a cardiac period value being indicative of a temporal length of a cardiac cycle, wherein the lower temporal deviation threshold is predefined such that it is equal to or larger than the temporal length of the cardiac cycle as indicated by the provided cardiac period value.

5. The ultrasound image generation system as defined in claim 1, wherein the ultrasound images combining unit (14) is adapted to combine provided intravascular ultrasound images which have been determined based on intravascular ultrasound signals acquired at acquisition locations (9, 10) which differ by less than a predefined spatial deviation threshold.

6. The ultrasound image generation system as defined in claim 5, wherein the ultrasound images combining unit (14) is adapted to provide a morphology of the vessel (6) and to modify the predefined spatial deviation threshold depending on the provided morphology.

7. The ultrasound image generation system as defined in claim 1, wherein the ultrasound images combining unit (14) is adapted to combine temporally adjacent intravascular ultrasound images.

8. The ultrasound image generation system as defined in claim 1, wherein the ultrasound image generation system (1) further comprises a controller (5) for controlling an ultrasound signals acquisition unit (7) for acquiring the intravascular ultrasound signals, while the ultrasound signals acquisition unit (7) is moved within and to different locations along the length of the vessel (6) by using a moving unit (17), wherein the pressure values providing unit (4, 5) is adapted to provide pressure values being indicative of the current pressure within the vessel (6) while the ultrasound signals acquisition unit (7) is moved, wherein the controller (5) is adapted to control the ultrasound signals acquisition unit (7) such that, for determining an intravascular ultrasound image, intravascular ultrasound signals are acquired, if a difference between a current pressure value and the reference pressure value being a pressure value provided for a last acquisition time at which intravascular ultrasound signals for determining a previous intravascular ultrasound image have been acquired, is smaller than the predefined pressure deviation threshold, wherein the ultrasound images providing unit is adapted to provide the intravascular ultrasound images by determining them based on the acquired ultrasound signals.

9. The ultrasound image generation system as defined in claim 8, wherein the controller (5) is adapted to control the ultrasound signals acquisition unit (7) and also the moving unit (17) such that the ultrasound signals are acquired at acquisition locations (9, 10) having a predetermined spatial resolution.

10. The ultrasound image generation system as defined in claim 9, wherein the controller (5) is adapted to control a) the moving unit (17) such that, after intravascular ultrasound signals have been acquired at an acquisition location, the ultrasound signals acquisition unit (7) is moved to a next acquisition location which corresponds to the predetermined spatial resolution, and b) the ultrasound signals acquisition unit (7) such that intravascular ultrasound signals are acquired at the next acquisition location if a current pressure differs by less than the predefined deviation threshold from the reference pressure value being a pressure value measured when acquiring the intravascular ultrasound signals at the previous acquisition location.

11. The ultrasound image generation system as defined in claim 1, wherein the ultrasound images combining unit (14) is adapted to generate several combined ultrasound images for different reference pressure values, wherein for generating a respective combined ultrasound image provided intravascular ultrasound images are combined, which have been determined based on intravascular ultrasound signals acquired at acquisition times (11, 12) at which the pressure values differ by less than a predefined deviation threshold from the respective reference pressure value, depending on the acquisition locations (9, 10).

12. The ultrasound image generation system as defined in claim 1, wherein the pressure values providing unit (4) is adapted to provide the intravascular pressure values such that they are indicative of the pressure within the vessel (6) at the acquisition locations (9, 10).

13. The ultrasound image generation system as defined in claim 1, wherein the ultrasound images providing unit (4, 5) and the pressure values providing unit (4) are integrated in a single unit.

14. An ultrasound image generation method for generating an intravascular ultrasound image, wherein the ultrasound image generation method comprises:
- providing intravascular ultrasound images of a vessel (6), which have been determined based on intravascular ultrasound signals acquired at different acquisition times (11, 12) and at different acquisition locations (9, 10) along the length of the vessel (6) by an ultrasound images providing unit (4, 5),
- providing the different acquisition locations, at which the intravascular ultrasound signals have been acquired, by an acquisition locations providing unit (18),
- providing intravascular pressure values being indicative of the pressure within the vessel (6) at the acquisition times (11, 12) by a pressure values providing unit (4),
- generating a combined ultrasound image by combining provided intravascular ultrasound images, which have been determined based on intravascular ultrasound signals acquired at acquisition times (11, 12) at which the pressure values differ by less than a predefined deviation threshold from a reference pressure value, depending on the acquisition locations (9, 10) by an ultrasound images combining unit (14).

15. A computer program for generating an intravascular ultrasound image, the computer program comprising program code means for causing an ultrasound image generation system as defined in claim 1 to carry out the steps of the ultrasound image generation method as defined in claim 14, when the computer program is run on a computer controlling the ultrasound image generation system.
